# EUROPÄISCHE PATENTSCHRIFT

(11) **EP 0 537 541 B1**
(45) Veröffentlichungstag und Bekanntmachung des Hinweises auf die Patenterteilung: **13.03.1996**
(21) Anmeldenummer: 92116540.3
(22) Anmeldetag: 28.09.1992
(51) Int. Cl.: C07C 209/72, C07C 209/16, C07C 211/35

(54) **Verfahren zur Herstellung eines Gemisches aus Cyclohexylamin und Dicyclohexylamin**
Process for the preparation of a mixture of cyclohexylamine and dicyclohexylamine
Procédé pour la préparation d'un mélange de cyclohexylamine et de dicyclohexylamine

(30) Priorität: 11.10.1991 DE 4133675
(43) Veröffentlichungstag der Anmeldung: 21.04.1993
(73) Patentinhaber: BAYER AG, D-51368 Leverkusen (DE)
(72) Erfinder: Immel, Otto, Dr., W-4150 Krefeld (DE); Buysch, Hans-Josef, Dr., W-4150 Krefeld (DE); Darsow, Gerhard, Dr., W-4150 Krefeld (DE)

(56) Entgegenhaltungen:
- EP-A- 0 053 817
- EP-A- 0 227 868
- EP-A- 0 324 984
- FR-A- 1 530 477
- FR-A- 2 115 860
- US-A- 2 571 016
- US-A- 3 351 661

## Beschreibung

Die vorliegende Erfindung betrifft ein Verfahren zur Herstellung von Cyclohexylamin und Dicyclohexylamin im Gemisch miteinander durch Umsetzung von Phenol mit Anilin und/oder Ammoniak unter hydrierenden Bedingungen in Gegenwart eines Palladium-Katalysators. Der Katalysator hat einen Träger aus Niobsäure, Tantalsäure oder einem Gemisch von Niobsäure und Tantalsäure oder einen diese Säuren enthaltenden Träger.

Eine wichtige Methode zur Herstellung von gegebenenfalls substituiertem Cyclohexylamin bzw. Dicyclohexylamin ist die Reduktion der entsprechenden aromatischen Nitroverbindungen (Houben-Weyl, Methoden der Org.Chemie, Band XI/1, S. 360 ff.) zu primären aromatischen Aminen und deren Kernhydrierung. Die Nitrierung verläuft jedoch häufig nicht einheitlich und liefert daher fast stets Isomerengemische.

Daneben besteht die Möglichkeit, Phenole durch teilweise Hydrierung in Cyclohexanone zu überführen und diese mit Ammoniak und Wasserstoff zu Cyclohexylaminen umzusetzen (DE-AS 1 124 487, DE-AS 1 298 098, DE-AS 1 144 267, US-PS 3.124.614, CH 463 493, DE-OS 2 045 882, Houben-Weyl, loc. cit., S.611-617).

Die genannten Herstellungswege erfordern mehrere voneinander unabhängige Verfahrensschritte und sind daher recht umständlich und nicht besonders wirtschaftlich.

Die einstufige direkte Überführung von nicht substituiertem Phenol mit Ammoniak und Wasserstoff in Cyclohexylamin gelingt nach bisheriger Kenntnis in Gegenwart von Ruthenium- oder Rhodiumkatalysatoren (JP-A 40/34 677, FR 1.427.543, GB 1.031.169, DE-AS 12 76 032). Für die bereits erwähnte Kernhydrierung von Anilin zu Cyclohexylamin wurden folgende Katalysatoren eingesetzt: Kobalt-Katalysatoren mit einem basischen Zusatz (GB 969 542), Raney-Kobalt (JP 68/03180), Ruthenium-Katalysatoren (DE-AS 1 106 319), mit Alkalimetallverbindungen dotierte Ruthenium-Katalysatoren (US 3.636.108) oder Nickel-Katalysatoren (DE-PS 805 518).

Die meisten der genannten Verfahren werden unter Druck betrieben und ergeben hauptsächlich Cyclohexylamin neben nur wenig Dicyclohexylamin. Das Dicyclohexylamin wird daher vielfach durch andere Verfahren hergestellt, beispielsweise durch Druckhydrierung von Diphenylamin unter Verwendung eines Ruthenium-Katalysators (DE-AS 1 106 319). Weiterhin entsteht Dicyclohexylamin bei der Umsetzung von Cyclohexanon mit Cyclohexylamin in Gegenwart eines Palladium/Kohle-Katalysators unter einem Wasserstoffdruck von etwa 4 bar (FR 1.333.692). Das Verfahren der genannten DE-PS 805 518 ist hauptsächlich auf die Gewinnung von Dicyclohexylamin ausgerichtet, arbeitet jedoch mit umständlichen Nebenproduktrückführungen. Dicyclohexylamin entsteht weiterhin bei der katalytischen Umsetzung von Phenol mit Wasserstoff und Ammoniak (US 3.351.661) sowie bei der Hydrierung eines Gemisches aus Anilin und Phenol (US 2.571.016). Dagegen entsteht Monocyclohexylamin aus Phenol, Ammoniak und Wasserstoff in Gegenwart eines auf einem Träger aufgebrachten Edelmetalls. (EP 53 817); dieser Katalysator enthält vorzugsweise einen basischen Stoff und/oder ein Element aus der Gruppe Ib oder IIb des Periodensystems der Elemente (Mendelejew). Mit einem Palladium/Kobalt-Katalysator auf Aluminiumspinell erhält man aus Phenol und Ammoniak in Gegenwart von Wasserstoff Anilin (EP 53 696).

Die Katalysatoren der genannten Verfahren haben eine unbefriedigende Standzeit; des weiteren werden zum Teil beträchtliche Mengen an Cyclohexan als wertloses Abfallprodukt gebildet. Es bestand daher der Wunsch, ein in technischem Maßstab brauchbares Verfahren zu entwickeln, bei welchem der Verlust durch die Bildung von Cyclohexan zurückgedrängt wird und die Standzeit des verwendeten Katalysators verbessert ist. Des weiteren bestand der Wunsch nach einem Verfahren, bei dem sowohl Cyclohexylamin und Dicyclohexylamin gebildet werden, deren Menge je nach dem Bedarf dieser Stoffe variabel eingestellt werden kann.

Weitere Verfahren, die zu Gemischen von Cyclohexylamin und Dicyclohexylamin führen, sind aus EP-A 0 324 984 und FR-A 1.530.477 bekannt. EP '984 beschreibt hierzu einen Katalysator, der obligatorisch zwei Platingruppenmetalle, nämlich Ru und Pd enthält; der Träger dieses Katalysators, beispielsweise Al₂O₃ oder Aluminiumspinell, ist durch Mitverwendung von Alkalimetallverbindungen alkalisch eingestellt. Anilin als Ausgangsstoff wird bei vermindertem bis mäßig erhöhtem Druck umgesetzt. In ER '477 wird ebenfalls Anilin bei vermindertem bis mäßig erhöhtem Druck umgesetzt. An einem Pd auf Al₂O₃, SiO₂, Silikoaluminaten oder Gemischen hiervon entsteht überwiegend Cyclohexylamin.

Es wurde überraschenderweise gefunden, daß die genannten Forderungen erreicht werden können, wenn man einen Palladium-Katalysator einsetzt, dessen Träger Niobsäure, Tantalsäure oder ein Gemisch beider ist oder diese Säuren enthält.

Die Erfindung betrifft daher ein Verfahren zur Herstellung eines Gemisches aus Cyclohexylamin und Dicyclohexylamin durch Umsetzung von Phenol mit Anilin, Ammoniak oder einem Gemisch von Anilin und Ammoniak in Gegenwart von Wasserstoff an einem Katalysator, das dadurch gekennzeichnet ist, daß ein Palladium-Katalysator mit einem Niobsäure- oder Tantalsäure- oder Niobsäure/Tantalsäure-Träger oder einen Träger, der diese Säuren enthält, eingesetzt wird, der 0,05-5 Gew.-% Palladium, bezogen auf das Gesamtgewicht des Katalysators, enthält, und bei 100-220°C und einem H₂-Partialdruck von 100-400 bar gearbeitet wird.

Der Palladiumgehalt des erfindungsgemäß einzusetzenden Katalysators beträgt bevorzugt 0,1-4 Gew.-%, besonders bevorzugt 0,1-3 Gew.-%, bezogen auf das Gesamtgewicht des Katalysators.

Die erfindungsgemäßen Katalysatoren sind demnach vor allem durch die Kombination von Palladium mit Niobsäure und/oder Tantalsäure als Träger oder auf dem Träger ausgezeichnet. Solche Katalysatoren liefern gegenüber den bekannten Trägerkatalysatoren höhere Ausbeuten an Dicyclohexylamin und zeichnen sich durch eine ausgezeichnete Standzeit aus.

Niobsäure ist bekanntlich ein Niobpentoxidhydrat (Nb₂O₅·nH₂0), das beispielsweise durch Behandlung wässriger Lösungen von Niobsäuresalzen mit starken Mineralsäuren oder durch Behandlung von Niobalkoholaten, Niobsäurehalogeniden oder Niobsäureestern mit Wasser, Säuren oder Basen erhalten werden kann. Derart gefällte Niobsäure wird getrocknet und stellt sodann eine schwerlösliche feste Verbindung dar, deren restlicher Wassergehalt nicht definiert ist, obwohl eine so hergestellte Niobsäure äußerlich wie ein trockenes Pulver erscheint. Die Herstellung der Niobsäure (Niobpentoxidhydrat) wird beipielsweise in Gmelins Handbuch der anorg. Chemie, 8. Auflage, Niob Teil B1, S.49, beschrieben.

Tantalsäure bzw. Tantalpentoxidhydrat (Ta₂O₅·nH₂O) für die Herstellung der erfindungsgemäß einzusetzenden Katalysatoren kann in analoger Weise durch Hydrolyse von Tantal(V)-Salzen, Tantal(V)-Alkoholaten oder anderen geeigneten hydrolysierbaren Tantal(V)-Verbindungen hergestellt werden. Die Hydrolyse vollzieht sich dabei in analoger Weise, wie dies oben für die Niobsäure beschrieben worden ist; sie ist beispielsweise in Gmelins Handbuch der anorg. Chemie, 8. Auflage, Tantal Teil B1, S. 53 (1970) und in Chem. Lett. 1988 S. 1573, beschrieben. Das für eines der beiden Elemente beschriebene gilt grundsätzlich auch für das jeweils andere. Die große chemische Ähnlichkeit beider Elemente und ihrer Verbindungen drückt sich auch darin aus, daß sie in ihrem natürlichen Vorkommen weitgehend miteinander vergesellschaftet sind.

In bevorzugter Weise wird als Träger Niobsäure eingesetzt, insbesondere solche, die einen aus der natürlichen Herkunft stammenden Anteil an Tantalsäure von 0,0001-10 Mol-%, bezogen auf die Summe der Molzahl von Niob- und Tantalsäure, enthält.

Damit die Niob- und/oder Tantalsäure in die für den Einsatz als Festbett-Katalysator günstige stückige Form gebracht werden kann, wird beipielsweise der feuchte Niederschlag der Hydrolyse in einem Kneter durchgeknetet und in einem Granulierapparat zu Formlingen verarbeitet. Die feuchten Formkörper werden anschließend beispielsweise bei 120°C getrocknet und 0,5-5 Stunden bei 200-400°C kalziniert.

Hierbei entsteht eine BET-Oberfläche von 5-350 m²/g. Zur Herstellung von Granulaten, Extrudaten oder Kugeln kann Niob- und/oder Tantalsäure auch mit einem Bindemittel verpreßt und granuliert werden.

Niob- bzw. Tanatalsäure ist bezüglich des erfindungsgemäßen Katalysators eine aktive Substanz, deren Aktivität auch durch Vermischen mit anderen Feststoffen erhalten bleibt. Geeignete Feststoffe sind beispielsweise Aluminiumoxid, Titandioxid, Zinkoxid, Magnesiumoxid, Eisenoxid, Siliziumdioxid, Graphit und andere. Diese Feststoffe können auch in der oben beschriebenen Weise als Bindemittel eingesetzt werden. Gemische der Niob- bzw. Tantalsäure mit diesen Feststoffen können im Verhältnis von 5:95-99:1, bevorzugt 50:50-98:2, eingesetzt werden. Bevorzugte erfindungsgemäß einsetzbare Katalysatoren enthalten Niob- bzw. Tantalsäure oder ein Gemisch beider ohne weiteren Zusatz. Für den Fall der Kombination mit einem der oben genannten Feststoffe erhält man einen besonders wirksamen Katalysatorträger, wenn man Al₂O₃-Granulat mit einer Lösung einer Niob- und/oder Tantalverbindung so imprägniert, daß der Gehalt an Niob- und/oder Tantalsäure des so hergestellten Trägers 0,2-30, vorzugsweise 0,5-10 Gew.-% beträgt.

Der Katalysator kann so hergestellt werden, daß man auf Niobsäure, Tantalsäure oder ein Gemisch beider oder auf einen der vorgenannten weiteren Träger, die Niob-und/oder Tantalsäure enthalten, Palladium in Form einer löslichen Verbindung auftränkt, nach dem Auftränken trocknet und den Katalysator in der dann vorliegenden Form oder nach einer Vorbehandlung mit Wasserstoff bei 120-400°C, bevorzugt bei 150-340°C, einsetzt. Diese H₂-Vorbehandlung wird in bevorzugter Weise in dem Reaktor vorgenommen, in dem anschließend das erfindungsgemäße Verfahren durchgeführt wird.

Der Katalysatorträger liegt hierzu in der Form von Pillen, Kugeln oder Bruchstücken mit Abmessungen von etwa 1-10 mm vor. Das Auftränken der Palladium-Verbindung geschieht in einer dem Fachmann grundsätzlich bekannten Weise. Das Trocknen wird beispielsweise bei 100-140°C und unter vermindertem bis normalen Druck, etwa bei 1-1 000 mbar, vorgenommen.

Die eingesetzten Palladiumverbindungen können in Wasser oder in geeigneten organischen Lösungsmitteln gelöst werden. Bevorzugt werden sie in organischen Lösungsmitteln gelöst, wie in einfachen Alkoholen, Ketonen, Nitrilen oder cyclischen Ethern. Beispiele für solche Lösungsmittel sind Methanol, Ethanol, Aceton, Acetonitril und Dioxan. Geeignete Palladiumverbindungen sind beispielsweise das Chlorid, Nitrat oder Acetat.

Die beschriebenen Katalysatoren können erfindungsgemäß in hervorragender Weise zur hydrierenden Umsetzung von gegebenenfalls substituiertem Anilin mit Phenol sowie zur hydrierenden Umsetzung eines Phenol-Ammoniak-Gemisches oder eines Dreiergemisches Phenol/Anilin/Ammoniak eingesetzt werden. Hierbei entsteht ein Gemisch von Cyclohexylamin und Dicyclohexylamin der Formeln 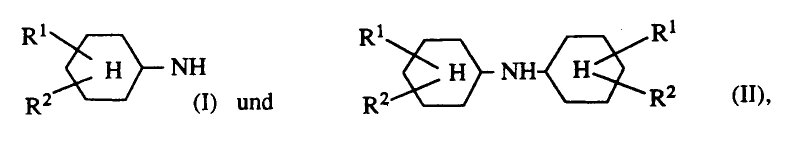
in denen
- R¹ und R²: unabhängig voneinander Wasserstoff, C₁-C₄-Alkyl oder C₁-C₄-Alkoxy bedeuten.

Cyclohexylamin und die Dicyclohexylamin weisen demnach das Substitutionsmuster des zugrundeliegendenden Phenols oder Anilins der Formeln 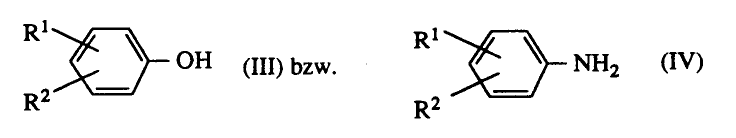
auf, in denen
- R¹ und R²: die genannte Bedeutung haben.

In besonders überraschender Weise läßt sich unter Verwendung der beschriebenen Katalysatoren in Abhängigkeit von der Hydriertemperatur die Menge des dabei entstehenden Dicyclohexylamins gegenüber der Menge des Monocyclohexylamins verändern, wodurch eine gezielte Herstellung von Dicyclohexylamin in größeren Mengen möglich ist. Beim Arbeiten im oberen Teil des erfindungsgemäßen Temperaturbereiches erhöht sich der Anteil des Monocyclohexylamins gegenüber dem des Dicyclohexylamins und umgekehrt.

Die genannten Alkyl- bzw. Alkoxysubstituenten sind beispielsweise: Methyl, Ethyl, Propyl, Isopropyl, Butyl, Isobutyl, Methoxy, Ethoxy, Propoxy, Isopropoxy, Butoxy oder Isobutoxy. In bevorzugter Weise haben die genannten Substituenten 1-2 C-Atome, besonders bevorzugt stellen sie Methyl bzw. Methoxy dar. In weiterhin bevorzugter Weise bedeutet R² Wasserstoff, während R¹ den genannten Bedeutungsumfang hat. In besonders bevorzugter Weise richtet sich das erfindungsgemäße Verfahren auf die Kernhydrierung von nicht substituiertem Anilin in Gegenwart von nicht substituiertem Phenol sowie auf die hydrierende Umsetzung eines Phenol/Ammoniak-Gemisches.

Das erfindungsgemäße Verfahren läßt sich in einem weiteren Druckbereich ausüben, der durch einen H₂-Partialdampfdruck von 100-400 bar, bevorzugt 150-350 bar, gekennzeichnet ist. Hierbei kann beispielsweise in der Rieselphase an fest angeordnetem Katalysator oder in einem Autoklaven gearbeitet werden. Dies eröffnet sowohl eine diskontinuierliche als auch eine kontinuierliche Arbeitsweise. Für technische Zwecke wird in bevorzugter Weise kontinuierlich, vorteilhaft in der Rieselphase, gearbeitet. Als Katalysatorbelastung wird eine Menge von 0,05-2 kg, bevorzugt 0,1-1 kg, besonders bevorzugt 0,15-0,6 kg Startmaterial pro Liter Katalysator und Stunde eingestellt.

Bei der Umsetzung von Phenol mit Anilin wird ein Molverhältnis beider Stoffe von 10:1-1:10 gewählt; das bevorzugte Molverhältnis beträgt 3:1-1:3. Bei der Umsetzung von Phenol und Ammoniak wird ein Molverhältnis von Phenol:Ammoniak = 1:20-1:1, bevorzugt 1:10-1:2, gewählt.

Das im erfindungsgemäßen Verfahren entstehende Reaktionsgemisch kann in üblicher Weise auf Cyclohexylamin und Dicyclohexylamin, beispielsweise durch Destillation, aufgetrennt werden. Nicht vollständig umgesetzte Ausgangsstoffe, wie Phenol bzw. Anilin, oder unvollständig hydrierte Reaktionsprodukte wie N-Cyclohexyl-anilin, können in einer dem Fachmann bekannten Weise recyclisiert werden.

Cyclohexylamine und Dicyclohexylamine des genannten Bedeutungsumfangs finden Verwendung zur Herstellung von Alterungsschutzmittel für Kautschuke und Kunststoffe, als Korrosionsschutzmittel, sowie als Vorprodukt für Pflanzenschutzmittel und Textilhilfsmittel.

### Beispiel 1

75 g Niobsäure, Nb₂O₅·nH₂O, die unter Zusatz von 3,5 % Graphitpulver zu 5 mm-Tabletten verformt worden waren, wurden mit einer Lösung getränkt, die aus 1,56 g Pd-Acetat und 26,8 g Acetonitril hergestellt worden war. Die auf diese Weise imprägnierten Niobsäuretabletten wurden 18 Stunden bei 100°C unter Wasserstrahlvakuum getrocknet. Danach wurde die Niobsäure nochmals auf die gleiche Weise mit 1 % Pd imgrägniert. 60 ml (61 g) des so hergestellten Katalysators wurden in ein senkrecht angeordnetes Druckrohr (Durchmesser 14 mm, Länge 70 cm) gebracht, das mit einem Ölthermostat beheizt wurde. Das Zwischenkornvolumen wurde mit feinem Seesand (0,2 - 0,4 mm) ausgefüllt. Der Katalysator wurde zunächst 3 Stunden bei 250°C und 270 bar mit Wasserstoff aktiviert, wobei stündlich 40 Liter Wasserstoff am unteren Ende des Reaktionsrohres entspannt wurden. Anschließend wurde bei 270 bar und 182°C mit der Hydrierung von Anilin im Gemisch mit Phenol begonnen, wobei das Anilin/Phenol-Gemisch und Wasserstoff von oben auf den Katalysator geleitet wurden. Die Flüssigkeit rieselte über den Katalysator nach unten in einen Abscheider. Am Kopf des Abscheiders wurden 26-62 Liter Wasserstoff entspannt. Die kontinuierlich eingespeiste Anilin/Phenol-Menge entsprach einer Katalysatorbelastung im Bereich von 0,2-0,3 g/ml Katalysator/h.

**Tabelle 1**

| (zu Beispiel 1): Hydrierende Umsetzung von Phenol und Anilin | | | | | | | |
|---|---|---|---|---|---|---|---|
| Belastung (g/ml·h) | T (°C) | Produkt (Gew.-%) | | Anilin | CA | NCHA | DCA |
| | | Anol | Phenol | | | | |
| 0,23 ¹⁾ | 160 | 2,3 | 13,3 | 18,2 | 2,0 | 8,2 | 54,5 |
| 0,24 ¹⁾ | 171 | - | 2,7 | 4,8 | 0,8 | 6,7 | 81,9 |
| 0,28 ¹⁾ | 196 | 0,7 | - | 0,1 | 6,7 | 0,3 | 92,0 |
| 0,28 ²⁾ | 206 | 5,8 | - | - | 2,1 | 0,1 | 91,6 |
| 0,22 ²⁾ | 208 | 4,4 | - | - | 2,6 | 0,1 | 92,1 |
| 0,19 ²⁾ | 231 | 7,4 | - | 0,1 | 17,3 | 0,1 | 74,0 |

| | | | | | | | |
|---|---|---|---|---|---|---|---|
| ¹⁾Anilin:Phenol = 2:1 molar; | | | | | | | |
| ²⁾Anilin:Phenol = 1:1 molar; Anol = Cyclohexanol; CA = Cyclohexylamin; NCHA = N-Cyclohexylanilin; DCA = Dicyclohexylamin | | | | | | | |

### Beispiel 2

400 g kugelförmiges γ-Al₂O₃ mit einem Durchmesser von 2-5 mm und einer spezifischen Oberfläche von 350 m²/g wurden mit einer Lösung von 23,3 g NbCl₅ in 120 g 37 %iger Salzsäure getränkt und dann bei 120°C getrocknet. Dann wurde der Katalysatorträger mit 410 g einer 16,9 Gew.-%igen wässrigen Ammoniaklösung getränkt und anschließend mit Wasser chloridfrei gewaschen und wieder getrocknet. 150 g des so hergestellten Katalysatorträgers wurden mit einer Lösung getränkt, die aus 3,13 g Pd-Acetat und 40 g Acetonitril hergestellt worden war. Nach erneutem Trocknen bei 120°C war der Katalysator einsatzbereit. Mit 60 ml (53 g) des so hergestellten Katalysators wurde die Umsetzung von Phenol mit NH₃ und Wasserstoff in einem Druckrohr der in Beispiel 1 eingesetzten Art vorgenommen. Zunächst wurde der Katalysator 3 Stunden bei 250°C und 270 bar im Wasserstoffstrom aktiviert. Über den aktivierten Katalysator wurden von oben pro Stunde 12-27 g Phenol und 4,6-5,8 g flüssiges Ammoniak eingeleitet, während bei einem ständigen, ebenfalls von oben nach unten geleiteten Zustrom von Wasserstoff der Druck in der Hydrierapparatur auf 280 bar gehalten wurde. Die Flüssigkeit rieselte über den Katalysator nach unten in einen Druckabscheider. Am Kopf des Abscheiders wurde Wasserstoff, der überschüssiges Ammoniak enthielt, entspannt (110-170 Liter/Stunde), um im Reaktionsrohr einen kontinuierlichen Gasstrom aufrecht zu erhalten. Die Apparatur wurde kontinuierlich betrieben, wobei die Reaktionstemperatur zwischen 135 und 215°C variiert wurde.

**Tabelle 2**

| (zu Beispiel 2): Hydrierende Umsetzung von Phenol mit Ammoniak | | | | | | | | |
|---|---|---|---|---|---|---|---|---|
| Phenol (g/h) | NH₃ (g/h) | T (°C) | Produkt (Gew.-%) | | Anilin | CA | NCHA | DCA |
| | | | Anol | Phenol | | | | |
| 15,4 | 5,8 | 135 | 8,7 | 0,5 | 0,5 | 22,5 | 0,2 | 67,8 |
| 12,1 | 5,5 | 145 | 1,0 | 0,3 | 0,1 | 30,1 | 0,1 | 68,3 |
| 27,5 | 5,0 | 200 | 5,1 | - | - | 21,9 | - | 73,0 |
| 22,2 | 5,5 | 215 | 5,9 | - | - | 22,7 | - | 71,4 |
| Anol, CA, NCHA und DCA wie in Tabelle 1 | | | | | | | | |

## Patentansprüche

1. Verfahren zur Herstellung eines Gemisches aus Cyclohexylamin und Dicyclohexylamin durch Umsetzung von Phenol mit Anilin, Ammoniak oder einem Gemisch von Anilin und Ammoniak in Gegenwart von Wasserstoff an einem Katalysator, dadurch gekennzeichnet, daß ein Palladium-Katalysator mit einem Niobsäure- oder Tantalsäure- oder Niobsäure/Tantalsäure-Träger oder einem Träger, der diese Säuren enthält, eingesetzt wird, der 0,05-5 Gew.-% Palladium, bezogen auf das Gesamtgewicht des Katalysators, enthält, und bei 100-220°C und einem H₂-Partialdruck von 100-400 bar gearbeitet wird.

2. Verfahren nach Anspruch 1, dadurch gekennzeichnet, daß bei einem H₂-Partialdruck von 150-350 bar gearbeitet wird.

3. Verfahren nach Anspruch 1, dadurch gekennzeichnet, daß Phenol und Anilin zur Umsetzung gebracht werden und ein Molverhältnis von 10:1-1:10 gewählt wird.

4. Verfahren nach Anspruch 3, dadurch gekennzeichnet, daß ein Molverhältnis Phenol:Anilin = 3:1-1:3 gewählt wird.

5. Verfahren nach Anspruch 1, dadurch gekennzeichnet, daß Phenol und Ammoniak zur Umsetzung gebracht werden und ein Molverhältnis von 1:20-1:1 gewählt wird.

6. Verfahren nach Anspruch 5, dadurch gekennzeichnet, daß ein Molverhältnis Phenol:NH₃ = 1:10-1:2 gewählt wird.

7. Verfahren nach Anspruch 1, dadurch gekennzeichnet, daß der Katalysator 0,1-4 Gew.-%, bevorzugt 0,1-3 Gew.-% Palladium, bezogen auf das Gesamtgewicht des Katalysators, enthält.

8. Verfahren nach Anspruch 1, dadurch gekennzeichnet, daß bei einer Katalysatorbelastung von 0,05-2 kg, bevorzugt 0,1-1 kg, besonders bevorzugt 0,15-0,6 kg Ausgangsprodukt pro Liter Katalysator pro Stunde gearbeitet wird.

9. Verfahren nach Anspruch 1, dadurch gekennzeichnet, daß der Katalysatorträger Al₂O₃ mit einem Gehalt von 0,2 bis 30 Gew.-%, bevorzugt 0,5 bis 10 Gew.-%, an Niob- und/oder Tantalsäure ist.

## Claims

1. Process for the preparation of a mixture of cyclohexylamine and dicyclohexylamine by reaction of phenol with aniline, ammonia or a mixture of aniline and ammonia in the presence of hydrogen over a catalyst, characterised in that a palladium catalyst which has a niobic acid or tantalic acid or niobic acid/tantalic acid support, or a support which contains these acids, and contains 0.05-5% by weight of palladium, based on the total weight of the catalyst, is employed and the reaction is carried out at 100-220°C under an H₂ partial pressure of 100-400 bar.

2. Process according to Claim 1, characterised in that the reaction is carried out under an H₂ partial pressure of 150-350 bar.

3. Process according to Claim 1, characterised in that phenol and aniline are reacted and a molar ratio of 10:1-1:10 is chosen.

4. Process according to Claim 3, characterised in that a molar ratio of phenol:aniline = 3:1-1:3 is chosen.

5. Process according to Claim 1, characterised in that phenol and ammonia are reacted and a molar ratio of 1:20-1:1 is chosen.

6. Process according to Claim 5, characterised in that a molar ratio of phenol:NH₃ = 1:10-1:2 is chosen.

7. Process according to Claim 1, characterised in that the catalyst contains 0.1-4% by weight, preferably 0.1-3% by weight, of palladium, based on the total weight of the catalyst.

8. Process according to Claim 1, characterised in that the reaction is carried out under a catalyst loading of 0.05-2 kg, preferably 0.1-1 kg, particularly preferably 0.15-0.6 kg, of starting product per litre of catalyst per hour.

9. Process according to Claim 1, characterised in that the catalyst support is Al₂O₃ with a content of 0.2 to 30% by weight, preferably 0.5 to 10% by weight, of niobic and/or tantalic acid.

## Revendications

1. Procédé de préparation d'un mélange d'une cyclohexylamine et d'une dicyclohexylamine par réaction d'un phénol avec une aniline, l'ammoniac ou un mélange d'une aniline et d'ammoniac, en présence d'hydrogène sur un catalyseur, caractérisé en ce qu'on utilise un catalyseur au palladium sur un support consistant en acide niobique ou acide tantalique ou acide niobique/acide tantalique ou sur un support contenant ces acides, avec 0,05 à 5% en poids de palladium, par rapport au poids total du catalyseur, et on opère à des températures de 100 à 220°C sous une pression partielle d'hydrogène de 100 à 400 bar.

2. Procédé selon la revendication 1, caractérisé en ce que l'on opère sous une pression partielle d'hydrogène de 150 à 350 bar.

3. Procédé selon la revendication 1, caractérisé en ce que l'on fait réagir le phénol et l'aniline à un rapport molaire de 10 : 1 à 1 : 10.

4. Procédé selon la revendication 3, caractérisé en ce que l'on opère à un rapport molaire phénol/aniline de 3 : 1 à 1 : 3.

5. Procédé selon la revendication 1, caractérisé en ce que l'on fait réagir le phénol et l'ammoniac à un rapport molaire de 1 : 20 à 1 : 1.

6. Procédé selon la revendication 5, caractérisé en ce que l'on opère à un rapport molaire phénol/NH₃ de 1 : 10 à 1:2.

7. Procédé selon la revendication 1, caractérisé en ce que le catalyseur contient 0,1 à 4% en poids, de préférence 0,1 à 3% en poids de palladium sur son poids total.

8. Procédé selon la revendication 1, caractérisé en ce que l'on opère avec une charge du catalyseur de 0,05 à 2 kg, de préférence de 0,1 à 1 kg, et plus spécialement de 0,15 à 0,6 kg de produit de départ par litre de catalyseur et par heure.

9. Procédé selon la revendication 1, caractérisé en ce que le support de catalyseur est Al₂O₃ contenant de 0,2 à 30% en poids, de préférence de 0,5 à 10% en poids, d'acide niobique et/ou d'acide tantalique.
